# EUROPEAN PATENT SPECIFICATION

(11) **EP 3 593 782 B1**
(45) Date of publication and mention of the grant of the patent: **12.07.2023**
(21) Application number: 17900009.6
(22) Date of filing: 22.09.2017
(51) Int. Cl.: A61H 3/00, A61F 5/02, A61H 1/02, A61F 5/01, A61F 2/64

(54) **ELBOW/KNEE JOINT ASSISTANCE DEVICE**
ELLENBOGEN-/KNIEGELENKUNTERSTÜTZUNGSVORRICHTUNG
DISPOSITIF D'AIDE À UNE ARTICULATION DE COUDE/DE GENOU

(30) Priority: 07.03.2017 JP 2017042317
(43) Date of publication of application: 15.01.2020
(73) Proprietor: Sakima Prosthetics&Orthotics Co., Ltd., Okinawa 9041201 (JP)
(72) Inventor: SAKIMA Tamotsu, Ginowan-shi Okinawa 901-2207 (JP); MATSUMOTO Hideo, Tokyo 160-8582 (JP)
(74) Representative: Körfer, Thomas
(86) International application number: PCT/JP2017/034189
(87) International publication number: WO 2018/163477

(56) References cited:
- WO-A1-93/22992
- WO-A1-2013/038709
- WO-A1-2015/084954
- WO-A1-2016/013039
- JP-A- 2000 005 247
- JP-A- 2007 160 029
- JP-A- 2017 035 372
- US-A1- 2011 098 618

## Description

### [TECHNICAL FIELD]

The present invention relates to a knee/elbow joint assist device which is mounted on a knee/elbow joint and used.

### [BACKGROUND TECHNIQUE]

The present inventor already proposed about a joint supporting device in which a user attaches the joint supporting device and can get down on one's knees in this state.

Patent document 1 proposes a joint supporting device provided with a connection member which connects and supports an inner supporting plate and an outer supporting plate to each other to surround a knee joint.

The patent document 1 proposes to make an essential structure member of the device of aluminum alloy.

### [PRIOR ART DOCUMENT]

### [Patent Document]

[Patent Document 1] Japanese Patent No.2903509 and Patent Documents WO2015084954A1, WO2016013039A1, US2011098618A1 WO9322992A1 disclose a knee joint assist device.

In particular US2011098618A1 discloses a knee joint assist device having a cable system that acts much like the body's natural ACL and MCL. The cables are routed around the knee joint in a way that resists the forces that cause excessive joint movement and injury to the ACL and or MCL. As the leg travels through the range of motion the cables provide external hyper extension, bending, and rotation support preventing the tibia bone from moving forward (hyper extending) or twisting (lateral rotation) and or laterally bending with respect to the femur.

### [SUMMARY OF THE INVENTION]

### [PROBLEM TO BE SOLVED BY THE INVENTION]

However, when one connection member as shown in the patent document 1 is used, it is necessary to limit movement of the connection member. It is possible to realize smooth movement by interlocking an upper arm and a lower arm to each other, but for that purpose, a structure for interlocking the upper arm and the lower arm to each other is required.

In an aluminum device as shown in the patent document 1, it is possible to adjust an arm shape and the connection member in accordance with symptoms of patients while utilizing elasticity of the aluminum. However, the shape of the aluminum arm is changed by a repeated load during usage, and an initial corrective force cannot be maintained. Therefore, it is desired to realize a device which can adjust a shape of an arm in accordance with a body shape and symptoms of a patient, and which is not plastically deformed by a repeated load during usage.

Thereupon, it is an object of the present invention to provide a knee/elbow joint assist device capable of limiting movement of a connection member and capable of preventing the connection member from drooping down.

Further, it is an object of the invention to provide a knee/elbow joint assist device capable of reducing a weight and enhancing fitness by integrally making one arm and the other arm of resin, capable of enhancing strength, and capable of limiting extending motion and bending motion with sufficient strength.

### [MEANS FOR SOLVING THE PROBLEM]

The present invention is direct to a knee/elbow joint assist device according to claim 1. Subsidiary aspects of the invention are provided in the dependent claims

### [EFFECT OF THE INVENTION]

According to the knee/elbow joint assist device of the present invention, it is possible to limit movement of the connection member, and prevent the connection member from drooping down.

Further, According to the knee/elbow joint assist device of the invention, it is possible to reduce a weight of the device and to enhance the fitness, and to enhance the strength by sliding the sliding portions having arcs of 180° or larger. Further, it is possible to limit extending motion and bending motion with sufficient strength.

### [BRIEF DESCRIPTION OF THE DRAWINGS]

Fig. 1 is a perspective view of a knee/elbow joint assist device according to one embodiment of the present invention;
Figs. 2 are explanatory diagrams of a rotation shaft of the knee/elbow joint assist device;
Fig. 3 is a perspective view of a knee/elbow joint assist device according to another embodiment of the invention;
Figs. 4 are explanatory diagrams of a link mechanism of the knee/elbow joint assist device;
Fig. 5 is a perspective view of a knee/elbow joint assist device according to another embodiment of the invention;
Fig. 6 is a perspective view of a knee/elbow joint assist device according to another embodiment of the invention;
Fig. 7 is a perspective view of the knee/elbow joint assist device as viewed from a direction which is different from that of Fig. 6;
Fig. 8 is a perspective view showing one arm, the other arm and a connection member in the knee/elbow joint assist device;
Figs. 9 are diagrams showing the one arm and the other arm in the knee/elbow joint assist device; and
Fig. 10 is a perspective view of a knee/elbow joint assist device according to another embodiment of the invention.

### [MODE FOR CARRYING OUT THE INVENTION]

According to a first aspect of the present invention, one connection member and the other connection member are used as connection members which support one inner arm and the other inner arm placed on an inner side of a knee/elbow joint, and one outer arm and the other outer arm placed on the other side of the knee/elbow joint at regular intervals, a central portion of the one connection member and a central portion of the other connection member are restrained by a connection-restraining member, thereby interlocking the one arm and the other arm to each other, movement of the connection member can be limited, and it is possible to prevent the connection member from drooping down.

According to a second aspect of the invention, three points, i.e., a connecting location with respect to the one arm of the one connection member, a connecting location with respect to the other arm of the other connection member, and a restraining location between the one connection member and the other connection member can form a triangle structure, a shape of the triangle is changed in accordance with extending motion and bending motion caused by the one arm and the other arm. Therefore, the one arm and the other arm can be interlocked.

According to a third aspect of the invention, an inner turning member and an outer turning member are made as rotation shafts. According to this, it is possible to realize a light-weighted knee/elbow joint assist device which has a simple structure and which cannot be recognized by other persons. In a case of such a device also, it is possible to sufficiently keep strength of the device, and to limit the movement of the connection member.

According to a fourth aspect of the invention, the inner turning member and the outer turning member are made as ling mechanisms. According to this, a force for vertically separating a knee/elbow joint from each other is applied by the link mechanisms, it is possible to realize a knee/elbow joint assist device which reduces a load bearing on the knee/elbow joint. In the case of such a device also, it is possible to sufficiently keep strength of the device, and to limit the movement of the connection member.

According to a fifth aspect of the invention, the inner turning member is made as a rotation shaft and the outer turning member is made as a link mechanism. According to this, a force for vertically separating the knee/elbow joint outward is applied by the link mechanism, it is possible to realize a knee/elbow joint assist device which reduces a load bearing on the knee/elbow joint by degenerative knee elbow joint symptom. In the case of such a device also, it is possible to sufficiently keep strength of the device, and to limit the movement of the connection member.

According to a sixth aspect of the invention, the outer turning member is made as a rotation shaft and the inner turning member is made as a link mechanism. According to this, a force for vertically separating the knee/elbow joint inward is applied by the link mechanism, it is possible to realize a knee/elbow joint assist device which reduces a load bearing on the knee/elbow joint by degenerative knee elbow joint symptom. In the case of such a device also, it is possible to sufficiently keep strength of the device, and to limit the movement of the connection member.

According to a seventh aspect of the invention, the one arm and the other arm are integrally formed together of resin. According to this, it is possible to reduce a weight of the device and to enhance the fitness, the sliding portions having arcs of 180° or more slide on each other, thereby enhancing the strength. Further, it is possible to limit the extending motion and the bending motion with sufficient strength.

According to an eighth aspect of the invention, the one arm and the other arm are integrally formed together of resin. According to this, it is possible to reduce a weight of the device and to enhance the fitness, the sliding portions having arcs of 180° or more slide on each other, thereby enhancing the strength. Further, it is possible to limit the extending motion and the bending motion with sufficient strength.

According to a ninth aspect of the invention, it is possible to limit the extending motion and the bending motion with sufficient strength.

### [Embodiments]

A knee/elbow joint assist device according to one embodiment of the present invention will be described using drawings.

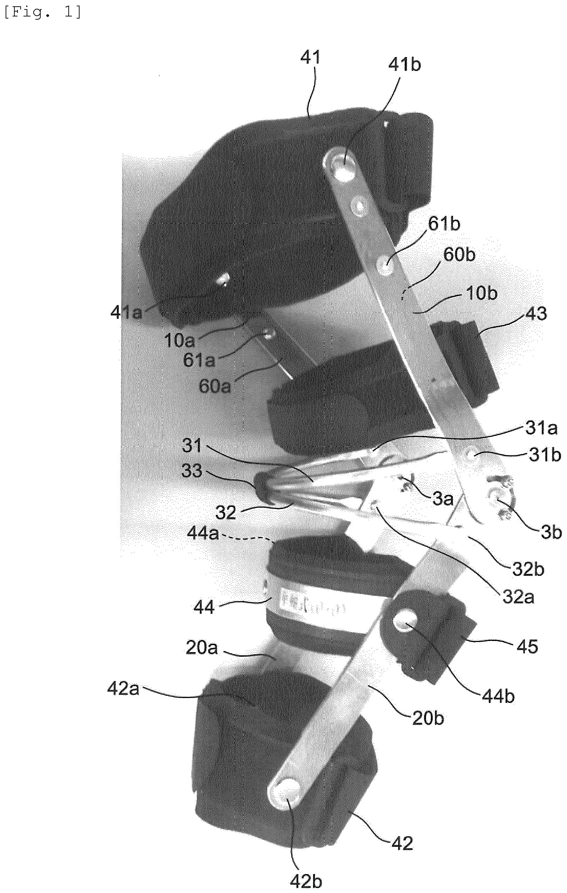

Fig. 1 is a perspective view of the knee/elbow joint assist device according to the embodiment.

The knee/elbow joint assist device of the embodiment includes one inner arm (one arm) 10b placed on one side of an inner side of a knee joint, the other inner arm (other arm) 20b placed on the other side of the inner side of the knee joint, one outer arm (one arm) 10a placed on one side of an outer side of the knee joint, the other outer arm (other arm) 20a placed on the other side of the outer side of the knee joint, and one connection member 31 and the other connection member 32 placed on a rear knee side.

One end of the one connection member 31 is turnably connected to the other end portion 31b of the one inner arm 10b, and the other end of the one connection member 31 is turnably connected to the other end portion 31a of the one outer arm 10a. One end of the other connection member 32 is turnably connected to one end portion 32b of the other inner arm 20b, and the other end of the other connection member 32 is turnably connected to the one end portion 32a of the other outer arm 20a.

The other end of the one inner arm 10b and the one end of the other inner arm 20b are turnably connected to each other through an inner rotation shaft (inner turning member) 3b. The other end of the one outer arm 10a and the one end of the other outer arm 20a are turnably connected to each other through an outer rotation shaft (outer turning member) 3a.

The other end portion 31b to which the one end of the one connection member 31 is connected is located closer to one end of the one inner arm 10b than the inner rotation shaft 3b, and the other end portion 31a to which the other end of the one connection member 31 is connected is located closer to the one end of the one outer arm 10a than the outer rotation shaft 3a. The one end portion 32b to which the one end of the other connection member 32 is connected is located closer to the other end of the other inner arm 20b than the inner rotation shaft 3b, and the one end portion 32a to which the other end of the other connection member 32 is connected is located closer to the other end of the other outer arm 20a than the outer rotation shaft 3a.

The one connection member 31 and the other connection member 32 restrain a central portion of the one connection member 31 and a central portion of the other connection member 32 through a connection-restraining member 33. The connection-restraining member 33 restrains the one connection member 31 and the other connection member 32 by winding cloth, resin or metal material such that the central portion of the one connection member 31 and the central portion of the other connection member 32 do not separate from each other more than a predetermined range. It is possible to use a hinge mechanism as the connection-restraining member 33. It is preferable that the one connection member 31 and the other connection member 32 turn within a predetermined range in the interlocking manner by meshing the central portion of the one connection member 31 and the central portion of the other connection member 32 with each other. By making the connection-restraining member 33 as a timing belt or a gear mechanism, it is possible to smoothly interlock the one connection member 31 and the other connection member 32.

The one connection member 31 has a C-shape, one end of the C-shape is turnably connected to the other end portion 31b of the one inner arm 10b, and the other end of the C-shape is turnably connected to the other end portion 31a of the one outer arm 10a.

The other connection member 32 has a C-shape, one end of the C-shape is turnably connected to the one end portion 32b of the other inner arm 20b, and the other end of the C-shape is turnably connected to the one end portion 32a of the other outer arm 20a.

The one connection member 31 and the other connection member 32 are connected to each other through the connection-restraining member 33 at an intermediate position of the C-shape.

A distance between the other end portion 31b to which the one end of the one connection member 31 is connected and the one end portion 32b to which the one end of the other connection member 32 is connected, as well as a distance between the other end portion 31a to which the other end of the one connection member 31 is connected and the one end portion 32a to which the other end of the other connection member 32 is connected are varied in accordance with an extended state and a bent state caused by the one arms 10a and 10b and the other arms 20a and 20b.

According to this embodiment, three points, i.e., a connected location (other end portions 31b and 31a) between the one arms 10a and 10b of the one connection member 31, a connected location (one end portions 32b and 32a) between the other arms 20a and 20b of the other connection member 32, and a restraining location (connection-restraining member 33) between the one connection member 31 and the other connection member 32 form a triangle structure. Since a shape of the triangle is changed in accordance with the extended state and the bent state caused by the one arms 10a and 10b and the other arms 20a and 20b, it is possible to interlock the one arms 10a and 10b and the other arms 20a and 20b.

A thigh band 41 which is wound around a thigh portion is mounted on the one end of the one outer arm 10a and the one end of the one inner arm 10b. A lower thigh band 42 which is wound around a lower thigh portion is mounted on the other end of the other outer arm 20a and the other end of the other inner arm 20b.

A suspension belt 43 is placed such that it is wound between the thigh portion and the knee joint.

A calf 44 which is placed at a calf portion located higher than a maximum bulging portion of the calf portion is mounted on the other outer arm 20a and the other inner arm 20b. A calf band 45 placed on the side of a tibia is mounted on the other outer arm 20a and the other inner arm 20b.

The one outer arm 10a includes a pin 41a for mounting the thigh band 41 on the one end, and the one inner arm 10b includes a pin 41b for mounting the thigh band 41 on the one end. The other outer arm 20a includes a pin 42a for mounting the lower thigh band 42 on the other end, and the other inner arm 20b includes a pin 42b for mounting the lower thigh band 42 on the other end.

The thigh band 41 is provided such that it can turn with respect to the one outer arm 10a through the pin 41a and such that the thigh band 41 can turn with respect to the one inner arm 10b through the pin 41b. The lower thigh band 42 is provided such that it can turn with respect to the other outer arm 20a through the pin 42a and such that the lower thigh band 42 can turn with respect to the other inner arm 20b through the pin 42b.

One end of an inner suspension arm 60b is mounted on an inner side of the one inner arm 10b through a pin 61b. The suspension belt 43 is mounted on the other end of the inner suspension arm 60b. The inner suspension arm 60b is formed of spring material such as a leaf spring, and the inner suspension arm 60b is provided such that it elastically deforms between the one inner arm 10b and a body.

One end of an outer suspension arm 60a is mounted on an inner side of the one outer arm 10a through a connecting portion 61a. The suspension belt 43 is mounted on the other end of the outer suspension arm 60a. The outer suspension arm 60a is formed of spring material such as a leaf spring, and provided such that it elastically deforms between the one outer arm 10a and a body.

The other outer arm 20a includes a pin 44a for mounting the calf 44 and the calf band 45 on the side closer to the one end than the pin 42a.

The calf 44 and the calf band 45 are provided such that they can turn with respect to the other outer arm 20a through the pin 44a.

The other inner arm 20b includes a pin 44b for mounting the calf 44 and the calf band 45 on the side closer to the one end than the pin 42b.

The calf 44 and the calf band 45 are provided such that they can turn with respect to the other inner arm 20b through the pin 44b.

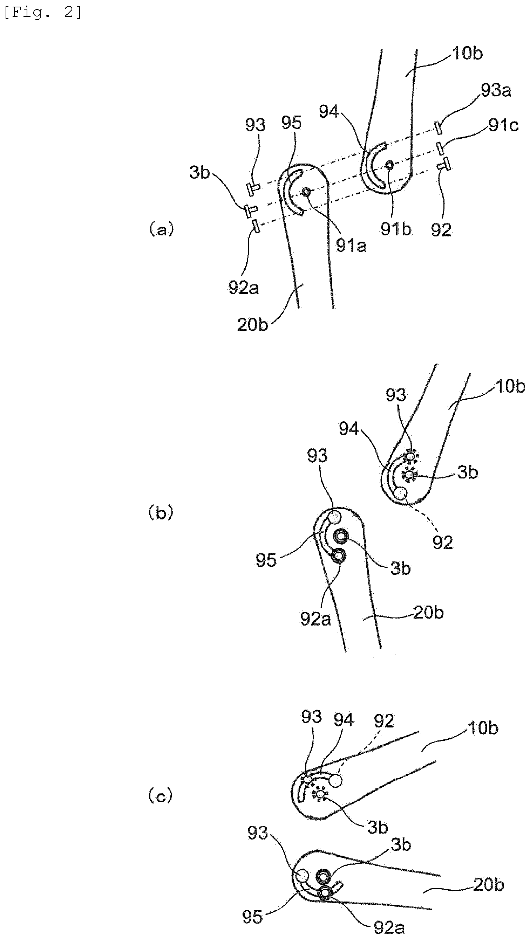

Figs. 2 are explanatory diagrams of the rotation shaft of the knee/elbow joint assist device according to this embodiment. In Figs. 2, the inner rotation shaft 3b will be described, but the outer rotation shaft 3a also has the same configuration.

As shown in Figs. 2, one inner arm projection 92 projecting toward the other inner arm 20b is provided on the other end of the one inner arm 10b, and the other inner arm projection 93 projecting toward the one inner arm 10b is provided on the one end of the other inner arm 20b.

One inner arm groove 94 on which the other inner arm projection 93 slides in an arc shape is provided in the other end of the one inner arm 10b, and the other inner arm groove 95 on which the one inner arm projection 92 slides in an arc shape is provided in the one end of the other inner arm 20b.

The inner rotation shaft 3b penetrates a hole 91a of the other inner arm 20b and a hole 91b of the one inner arm 10b, and is provided at its end with a fastening material 91c.

The one inner arm projection 92 penetrates the one inner arm groove 94 and the other inner arm groove 95, and is provided at its end with a fastening material 92a. The one inner arm projection 92 does not slide in the one inner arm groove 94.

The other inner arm projection 93 penetrates the other inner arm groove 95 and the one inner arm groove 94, and is provided at its end with a fastening material 93a. The other inner arm projection 93 does not slide in the other inner arm groove 95.

Fig. 2 (b) shows an extended state and Fig. 2 (c) shows a bent state. The one inner arm 10b and the other inner arm 20b turn around the inner rotation shaft 3b, the one inner arm projection 92 slides in the other inner arm groove 95, and the other inner arm projection 93 slides in the one inner arm groove 94.

As shown in the embodiment, the other inner arm projection 93 and the one inner arm groove 94 abut against each other, and the one inner arm projection 92 and the other inner arm groove 95 abut against each other. According to this, it is possible to enhance the rigidity as compared with a case where the other end of the one inner arm 10b and the one end of the other inner arm 20b are connected to each other only through the inner rotation shaft 3b.

A knee/elbow joint assist device according to another embodiment of the present invention will be described using the drawings.

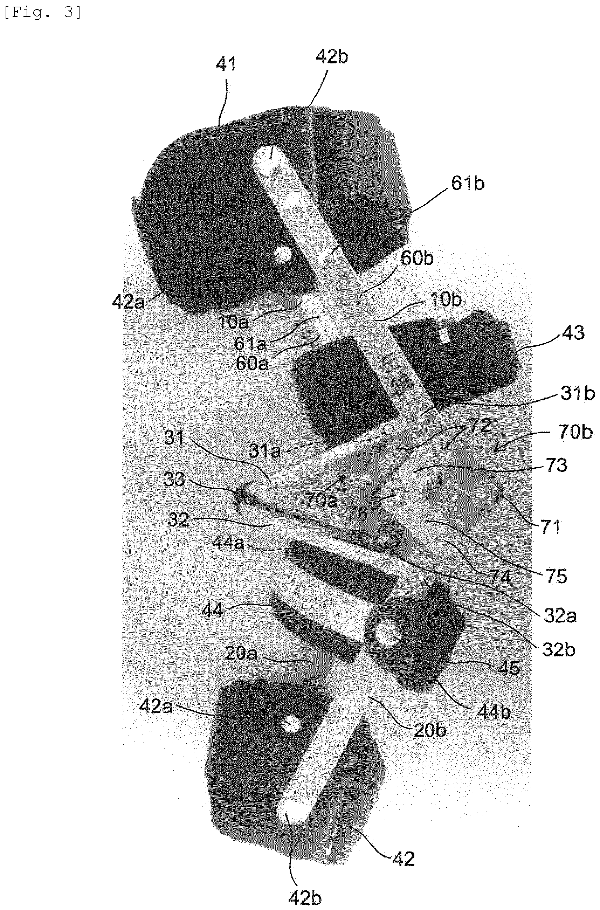

Fig. 3 is a perspective view of the knee/elbow joint assist device according to this embodiment.

The knee/elbow joint assist device of the embodiment includes the one inner arm 10b placed on one side of an inner side of a knee joint, the other inner arm 20b placed on the other side of the inner side of the knee joint, the one outer arm 10a placed on one side of an outer side of the knee joint, the other outer arm 20a placed on the other side of the outer side of the knee joint, and the one connection member 31 and the other connection member 32 placed on the side of the rear knee portion.

The one connection member 31 turnably connects its one end to the other end portion 31b of the one inner arm 10b, and turnably connects its other end to the other end portion 31a of the one outer arm 10a. The other connection member 32 turnably connects its one end to the one end portion 32b of the other inner arm 20b, and turnably connects its other end to the one end portion 32a of the other outer arm 20a.

The other end of the one inner arm 10b and the one end of the other inner arm 20b are turnably connected to each other through an inner link mechanism (inner turning member) 70b. The other end of the one outer arm 10a and the one end of the other outer arm 20a are turnably connected to each other through an outer link mechanism (outer turning member) 70a.

The one connection member 31 and the other connection member 32 restrain a central portion of the one connection member 31 and a central portion of the other connection member 32 through the connection-restraining member 33. The connection-restraining member 33 restrains the one connection member 31 and the other connection member 32 by winding cloth, resin or metal material such that the central portion of the one connection member 31 and the central portion of the other connection member 32 do not separate from each other more than a predetermined range. It is possible to use a hinge mechanism as the connection-restraining member 33. It is preferable that the one connection member 31 and the other connection member 32 turn within a predetermined range in the interlocking manner by meshing the central portion of the one connection member 31 and the central portion of the other connection member 32 with each other. By making the connection-restraining member 33 as a timing belt or a gear mechanism, it is possible to smoothly interlock the one connection member 31 and the other connection member 32.

The inner link mechanism 70b is composed of an inner front joint 71 which turnably connects the other end of the one inner arm 10b and the one end of the other inner arm 20b to each other, one inner link material 73 which turnably provides its one end on the other end side of the one inner arm 10b through an one inner joint 72, an other inner link material 75 which turnably provides its other end on the one end side of the other inner arm 20b through an other inner joint 74, and an inner rear joint 76 which turnably connects the other end of the one inner link material 73 and the one end of the other inner link material 75 to each other.

The outer link mechanism 70a is composed of an outer front joint 71 which turnably connects the other end of the one outer arm 10a and the one end of the other outer arm 20a to each other, an one outer link material 73 which turnably provides its one end on the other end side of the one outer arm 10a through an one outer joint 72, an other outer link material 75 which turnably provides its other end on the one end side of the other outer arm 20a through an other outer joint 74, and an outer rear joint 76 which turnably connects the other end of the one outer link material 73 and the one end of the other outer link material 75 to each other.

The one end of the one connection member 31 is turnably connected to the other end portion 31b of the one inner arm 10b on the side closer to the one end of the one inner arm 10b than the one inner joint 72 and the other end of the one connection member 31 is turnably connected to the other end portion 31a of the one outer arm 10a on the side closer to the one end of the one outer arm 10a than the one outer joint 72. The one end of the other connection member 32 is turnably connected to the one end portion 32b of the other inner arm 20b on the side closer to the other end of the other inner arm 20b than the other inner joint 74, and the other end of the other connection member 32 is turnably connected to the one end portion 32a of the other outer arm 20a on the side closer to the other end of the other outer arm 20a than the other outer joint 74.

The one connection member 31 has a C-shape, one end of the C-shape is turnably connected to the other end portion 31b of the one inner arm 10b on the side closer to the one end than the one inner joint 72, and the other end of the C-shape is turnably connected to the other end portion 31a of the one outer arm 10a on the side closer to the one end than the one outer joint 72.

The other connection member 32 has a C-shape, one end of the C-shape is turnably connected to the one end portion 32b of the other inner arm 20b on the side closer to the other end than the other inner joint 74, and the other end of the C-shape is turnably connected to the one end portion 32a of the other outer arm 20a on the side closer to the other end than the other outer joint 74.

The one connection member 31 and the other connection member 32 are connected to each other through the connection-restraining member 33 at an intermediate position of the C-shape.

A distance between the other end portion 31b to which the one end of the one connection member 31 is connected and the one end portion 32b to which the one end of the other connection member 32 is connected, as well as a distance between the other end portion 31a to which the other end of the one connection member 31 is connected and the one end portion 32a to which the other end of the other connection member 32 is connected are varied in accordance with an extended state and a bent state caused by the one arms 10a and 10b and the other arms 20a and 20b.

According to this embodiment, three points, i.e., a connected location (other end portions 31b and 31a) between the one arms 10a and 10b of the one connection member 31, a connected location (one end portions 32b and 32a) between the other arms 20a and 20b of the other connection member 32, and a restraining location (connection-restraining member 33) between the one connection member 31 and the other connection member 32 form a triangle structure. Since a shape of the triangle is changed in accordance with the extended state and the bent state caused by the one arms 10a and 10b and the other arms 20a and 20b, it is possible to interlock the one arms 10a and 10b and the other arms 20a and 20b.

A thigh band 41 which is wound around a thigh portion is mounted on the one end of the one outer arm 10a and the one end of the one inner arm 10b. A lower thigh band 42 which is wound around a lower thigh portion is mounted on the other end of the other outer arm 20a and the other end of the other inner arm 20b.

A suspension belt 43 is placed such that it is wound between the thigh portion and the knee joint.

A calf 44 which is placed at a calf portion located higher than a maximum bulging portion of the calf portion is mounted on the other outer arm 20a and the other inner arm 20b. A calf band 45 placed on the side of a tibia is mounted on the other outer arm 20a and the other inner arm 20b.

The one outer arm 10a includes a pin 41a for mounting the thigh band 41 on the one end, and the one inner arm 10b includes a pin 41b for mounting the thigh band 41 on the one end. The other outer arm 20a includes a pin 42a for mounting the lower thigh band 42 on the other end, and the other inner arm 20b includes a pin 42b for mounting the lower thigh band 42 on the other end.

The thigh band 41 is provided such that it can turn with respect to the one outer arm 10a through the pin 41a and such that the thigh band 41 can turn with respect to the one inner arm 10b through the pin 41b. The lower thigh band 42 is provided such that it can turn with respect to the other outer arm 20a through the pin 42a and such that the lower thigh band 42 can turn with respect to the other inner arm 20b through the pin 42b.

One end of an inner suspension arm 60b is mounted on an inner side of the one inner arm 10b through a pin 61b. The suspension belt 43 is mounted on the other end of the inner suspension arm 60b. The inner suspension arm 60b is formed of spring material such as a leaf spring, and the inner suspension arm 60b is provided such that it elastically deforms between the one inner arm 10b and a body.

One end of an outer suspension arm 60a is mounted on an inner side of the one outer arm 10a through a connecting portion 61a. The suspension belt 43 is mounted on the other end of the outer suspension arm 60a. The outer suspension arm 60a is formed of spring material such as a leaf spring, and provided such that it elastically deforms between the one outer arm 10a and a body.

The other outer arm 20a includes a pin 44a for mounting the calf 44 and the calf band 45 on the side closer to the one end than the pin 42a.

The calf 44 and the calf band 45 are provided such that they can turn with respect to the other outer arm 20a through the pin 44a.

The other inner arm 20b includes a pin 44b for mounting the calf 44 and the calf band 45 on the side closer to the one end than the pin 42b.

The calf 44 and the calf band 45 are provided such that they can turn with respect to the other inner arm 20b through the pin 44b.

In this embodiment, the one end of the one connection member 31 is connected to the one end of the one inner arm 10b through the one inner joint 72, and the other end of the one connection member 31 is connected to the one end of the one outer arm 10a through the one inner joint 72, but the one end of the one connection member 31 may be connected to the one inner joint 72 and the other end of the one connection member 31 may be connected to the one outer joint 72. Further, the one end of the other connection member 32 is connected to the other end of the other inner arm 20b through the other inner joint 74 and the other end of the other connection member 32 is connected to the other end of the other outer arm 20a through the other outer joint 74, but the one end of the other connection member 32 may be connected to the other inner joint 74 and the other end of the other connection member 32 may be connected to the other outer member 74.

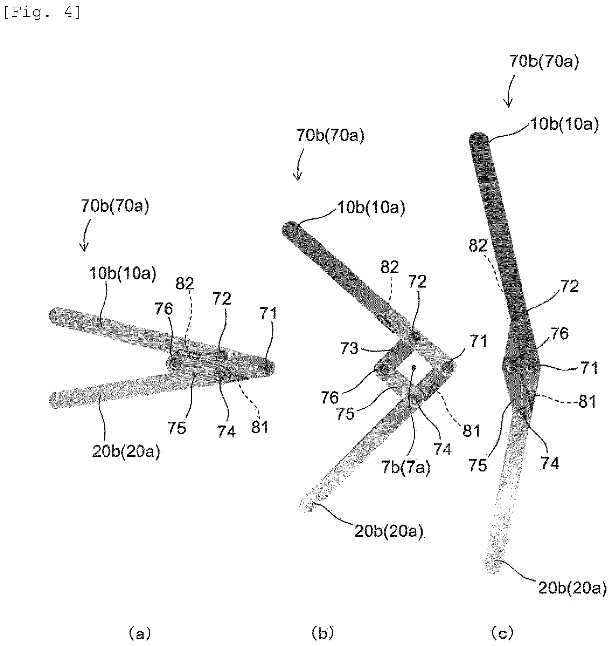

Figs. 4 are explanatory diagrams of the link mechanism of the knee/elbow joint assist device according to this embodiment, wherein Fig. 4(a) is a side view of the link mechanism in the bent state, Fig. 4(b) is a side view of the link mechanism in a state where it is slightly bent, and Fig. 4(c) is a side view of the link mechanism in the extended state.

As shown in Fig. 4(b), a virtual inner rotation shaft 7b in the inner link mechanism 70b is located at an intersection point between a virtual line which connects the inner front joint 71 and the inner rear joint 76 to each other and a virtual line which connects the one inner joint 72 and the other inner joint 74 to each other. A virtual outer rotation shaft 7a in the outer link mechanism 70a is located at an intersection point between a virtual line which connects the outer front joint 71 and the outer rear joint 76 to each other and a virtual line which connects the one inner joint 72 and the other inner joint 74 to each other.

According to the knee/elbow joint assist device of the embodiment, the other inner arm 20b is provided with an angle-restricting member 81 which abuts against the other inner link material 75 in the extended state for example. According to this, it is possible to restrict the extending angle. Further, the one inner arm 10b is provided with an angle-restricting member 82 which abuts against the other inner link material 75 in the bent state. According to this, it is possible to restrict the bending angle.

According to the knee/elbow joint assist device of the embodiment, it is possible to easily provide the one inner arm 10b, the other inner arm 20b, the one inner link material 73 and the other inner link material 75 with the angle-restricting member 81 or 82 which restricts the extending angle or the bending angle, and the angle-restricting members 81 and 82 can be provided at positions separated away from the knee joint. Therefore, it is possible to bear a large load.

The one inner link material 73 and the other inner arm 20b are placed in parallel to each other on the same plane, and the other inner link material 75 and the one inner arm 10b can be placed in parallel to each other on the same plane. Therefore, it is possible to enhance the strength of the inner link mechanism 70b.

A knee/elbow joint assist device of another embodiment of the present invention will be described using the drawings.

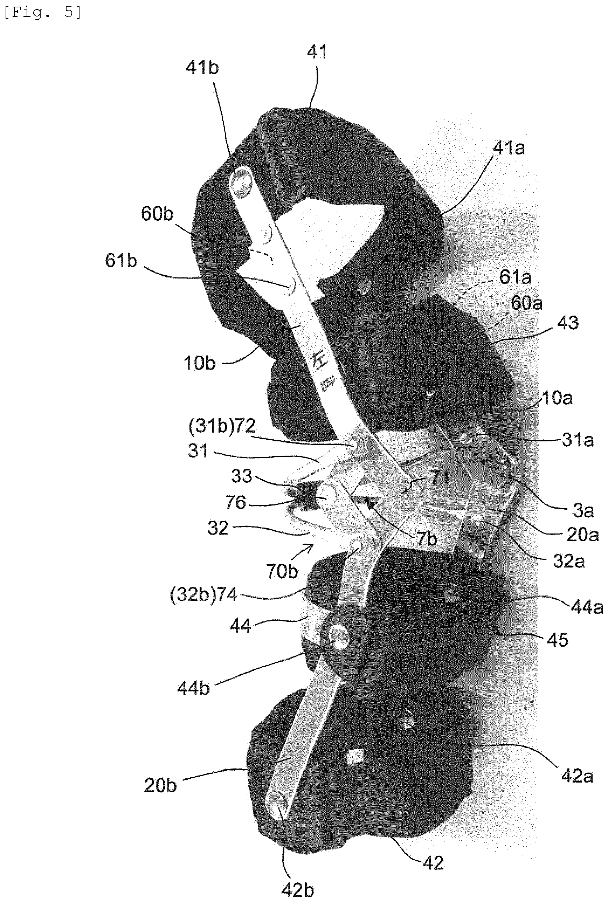

Fig. 5 is a perspective view of the knee/elbow joint assist device of this embodiment.

The knee/elbow joint assist device of this embodiment includes the one inner arm (one arm) 10b placed on one side of an inner side of a knee joint, the other inner arm (other arm) 20b placed on the other side of the inner side of the knee joint, one outer arm (one arm) 10a placed on one of measuring sides of the knee joint, the other outer arm (other arm) 20a placed on the other measuring side of the knee joint, and the one connection member 31 and the other connection member 32 placed on the rear knee portion side.

The other end of the one inner arm 10b and the one end of the other inner arm 20b are turnably connected to each other through the inner link mechanism (inner turning member) 70b. The other end of the one outer arm 10a and the one end of the other outer arm 20a are turnably connected to each other through the outer rotation shaft (outer turning member) 3a.

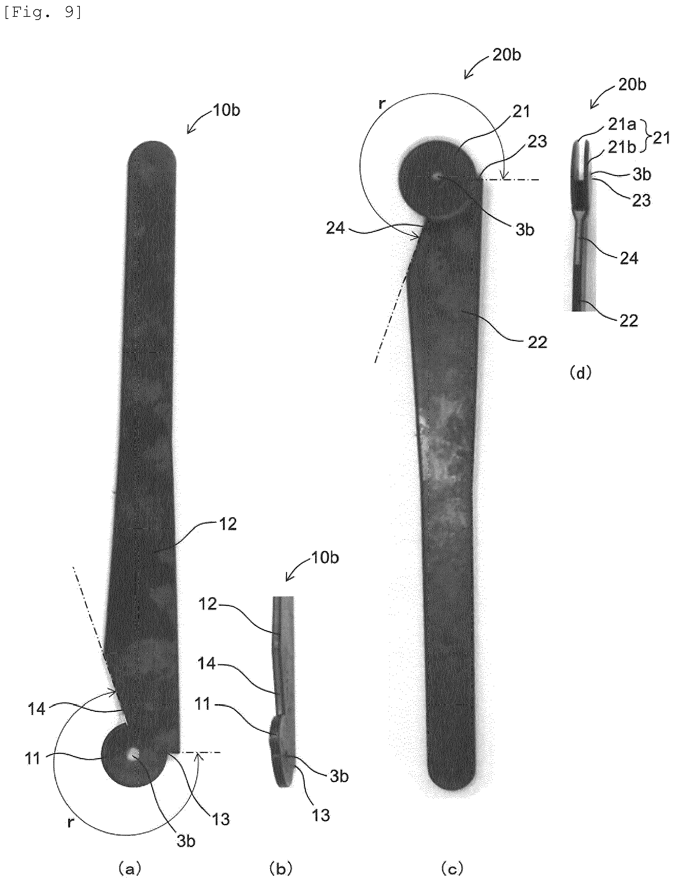

The link mechanism described using Figs. 4 can be applied to the inner link mechanism 70b. The rotation shaft described using Figs. 2 can be applied to the outer rotation shaft 3a. The rotation shaft shown in Figs. 9 can be applied to the outer rotation shaft 3a.

The one connection member 31 and the other connection member 32 restrain a central portion of the one connection member 31 and a central portion of the other connection member 32 through a connection-restraining member 33. The connection-restraining member 33 restrains the one connection member 31 and the other connection member 32 by winding cloth, resin or metal material such that the central portion of the one connection member 31 and the central portion of the other connection member 32 do not separate from each other more than a predetermined range. It is possible to use a hinge mechanism as the connection-restraining member 33. It is preferable that the one connection member 31 and the other connection member 32 turn within a predetermined range in the interlocking manner by meshing the central portion of the one connection member 31 and the central portion of the other connection member 32 with each other. By making the connection-restraining member 33 as a timing belt or a gear mechanism, it is possible to smoothly interlock the one connection member 31 and the other connection member 32.

The one end of the one connection member 31 is turnably connected to the other end portion 31b (one inner joint 72) of the one inner arm 10b, and the other end of the one connection member 31 is turnably connected to the other end portion 31a of the one outer arm 10a on the side closer to the one end of the one outer arm 10a than the outer rotation shaft 3a. The one end of the other connection member 32 is turnably connected to the one end portion 32b (other inner joint 74) of the other inner arm 20b, and the other end of the other connection member 32 is turnably connected to the one end portion 32a of the other outer arm 20a on the side closer to the other end than the outer rotation shaft 3a.

The one connection member 31 has a C-shape, one end of the C-shape is turnably connected to the other end portion 31b of the one inner arm 10b through the one inner joint 72, and the other end of the C-shape is turnably connected to the other end portion 31a of the one outer arm 10a.

The other connection member 32 has a C-shape, one end of the C-shape is connected to the one end portion 32b of the other inner arm 20b through the other inner joint 74, and the other end of the C-shape is turnably connected to the one end portion 32a of the other outer arm 20a.

The one connection member 31 and the other connection member 32 are connected to each other through the connection-restraining member 33 at an intermediate position of the C-shape.

A distance between the other end portion 31b to which the one end of the one connection member 31 is connected and the one end portion 32b to which the one end of the other connection member 32 is connected, as well as a distance between the other end portion 31a to which the other end of the one connection member 31 is connected and the one end portion 32a to which the other end of the other connection member 32 is connected are varied in accordance with an extended state and a bent state caused by the one arms 10a and 10b and the other arms 20a and 20b.

According to this embodiment, three points, i.e., a connected location (other end portions 31b and 31a) between the one arms 10a and 10b of the one connection member 31, a connected location (one end portions 32b and 32a) between the other arms 20a and 20b of the other connection member 32, and a restraining location (connection-restraining member 33) between the one connection member 31 and the other connection member 32 form a triangle structure. Since a shape of the triangle is changed in accordance with the extended state and the bent state caused by the one arms 10a and 10b and the other arms 20a and 20b, it is possible to interlock the one arms 10a and 10b and the other arms 20a and 20b.

A thigh band 41 which is wound around a thigh portion is mounted on the one end of the one outer arm 10a and the one end of the one inner arm 10b. A lower thigh band 42 which is wound around a lower thigh portion is mounted on the other end of the other outer arm 20a and the other end of the other inner arm 20b.

A suspension belt 43 is placed such that it is wound between the thigh portion and the knee joint.

A calf 44 which is placed at a calf portion located higher than a maximum bulging portion of the calf portion is mounted on the other outer arm 20a and the other inner arm 20b. A calf band 45 placed on the side of a tibia is mounted on the other outer arm 20a and the other inner arm 20b.

The one outer arm 10a includes a pin 41a for mounting the thigh band 41 on the one end, and the one inner arm 10b includes a pin 41b for mounting the thigh band 41 on the one end. The other outer arm 20a includes a pin 42a for mounting the lower thigh band 42 on the other end, and the other inner arm 20b includes a pin 42b for mounting the lower thigh band 42 on the other end.

The thigh band 41 is provided such that it can turn with respect to the one outer arm 10a through the pin 41a and such that the thigh band 41 can turn with respect to the one inner arm 10b through the pin 41b. The lower thigh band 42 is provided such that it can turn with respect to the other outer arm 20a through the pin 42a and such that the lower thigh band 42 can turn with respect to the other inner arm 20b through the pin 42b.

One end of an inner suspension arm 60b is mounted on an inner side of the one inner arm 10b through a pin 61b. The suspension belt 43 is mounted on the other end of the inner suspension arm 60b. The inner suspension arm 60b is formed of spring material such as a leaf spring, and the inner suspension arm 60b is provided such that it elastically deforms between the one inner arm 10b and a body.

One end of an outer suspension arm 60a is mounted on an inner side of the one outer arm 10a through a connecting portion 61a. The suspension belt 43 is mounted on the other end of the outer suspension arm 60a. The outer suspension arm 60a is formed of spring material such as a leaf spring, and provided such that it elastically deforms between the one outer arm 10a and a body.

The other outer arm 20a includes a pin 44a for mounting the calf 44 and the calf band 45 on the side closer to the one end than the pin 42a.

The calf 44 and the calf band 45 are provided such that they can turn with respect to the other outer arm 20a through the pin 44a.

The other inner arm 20b includes a pin 44b for mounting the calf 44 and the calf band 45 on the side closer to the one end than the pin 42b.

The calf 44 and the calf band 45 are provided such that they can turn with respect to the other inner arm 20b through the pin 44b.

According to the knee/elbow joint assist device of this embodiment, by using the inner link mechanism 70b where the four joints 71, 72, 74 and 76 are placed, the one inner joint 72 located at a position of the one inner arm 10b moves upward (toward one end) as the knee joint moves from the bent state to the extended state, and the other inner joint 74 located at a position of the other inner arm 20b moves downward (toward other end). Therefore, a force to vertically separate is applied to an inner side of the knee joint by the inner link mechanism 70b at the time of the extending motion. Since the force to vertically separate the inner side of the knee joint is applied by the inner link mechanism 70b at the time of the extending motion, it is possible to reduce a load bearing on the knee joint, pain of the knee joint is subdued and a patient is encouraged to walk.

Shaking of the outer side of the knee joint at the time of the extending motion and the bending motion is not generated by using the single outer rotation shaft 3a. Therefore, the force to vertically separate which is applied to the inner side of the knee joint can reliably be applied.

A knee/elbow joint assist device according to another embodiment of the invention will be described below.

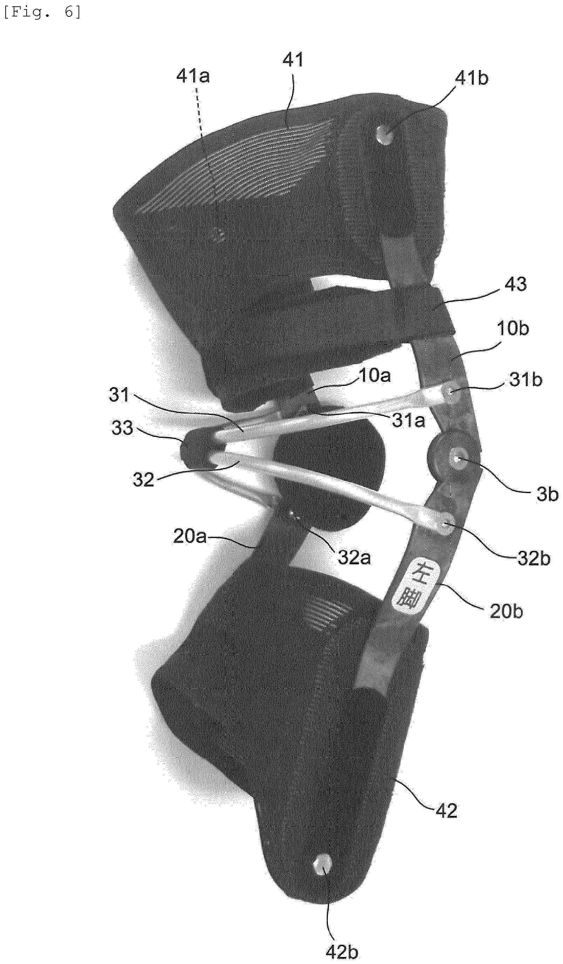

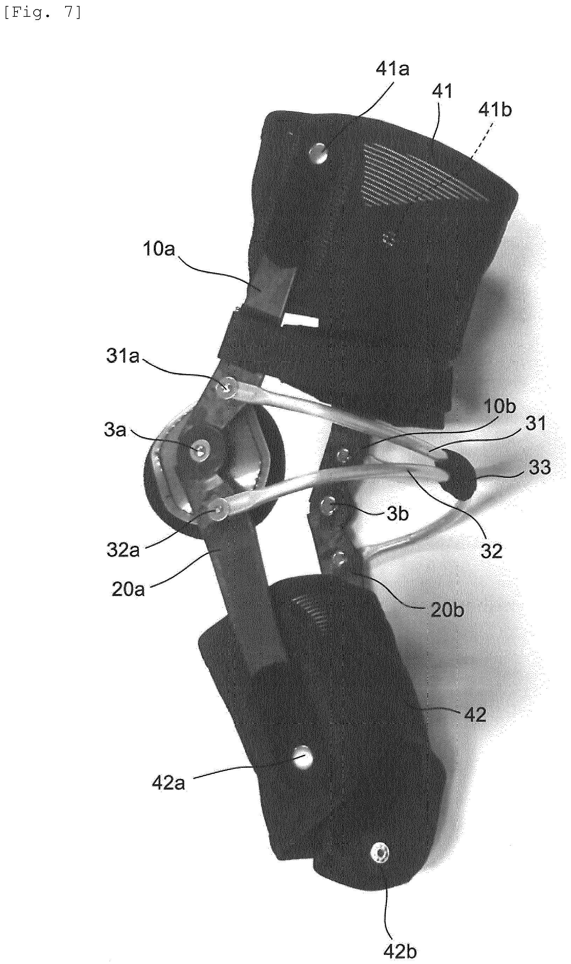

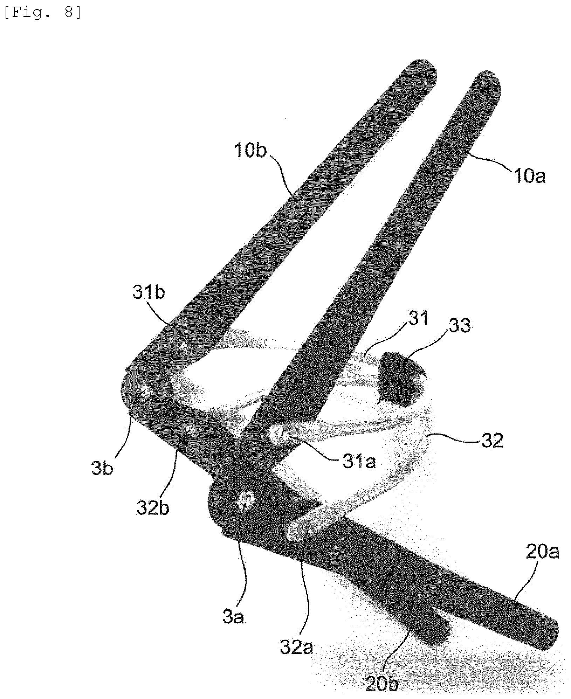

Fig. 6 is a perspective view of the knee/elbow joint assist device according to the other embodiment, Fig. 7 is a perspective view of the knee/elbow joint assist device as viewed from a direction which is different from that of Fig. 6, Fig. 8 is a perspective view showing one arm, the other arm and a connection member in the knee/elbow joint assist device, and Figs. 9 are diagrams showing the one arm and the other arm in the knee/elbow joint assist device.

The knee/elbow joint assist device of the embodiment includes one inner arm (one arm) 10b placed on one side of an inner side of a knee joint, the other inner arm (other arm) 20b placed on the other side of the inner side of the knee joint, one outer arm (one arm) 10a placed on one side of an outer side of the knee joint, the other outer arm (other arm) 20a placed on the other side of the outer side of the knee joint, and one connection member 31 and the other connection member 32 placed on a rear knee side.

One end of the one connection member 31 is turnably connected to the other end portion 31b of the one inner arm 10b, and the other end of the one connection member 31 is turnably connected to the other end portion 31a of the one outer arm 10a. One end of the other connection member 32 is turnably connected to one end portion 32b of the other inner arm 20b, and the other end of the other connection member 32 is turnably connected to the one end portion 32a of the other outer arm 20a.

The other end of the one inner arm 10b and the one end of the other inner arm 20b are turnably connected to each other through an inner rotation shaft (inner turning member) 3b. The other end of the one outer arm 10a and the one end of the other outer arm 20a are turnably connected to each other through an outer rotation shaft (outer turning member) 3a.

The one end of the one connection member 31 is connected on the side closer to the one end than the inner rotation shaft 3b, and the other end of the one connection member 31 is connected on the side closer to the one end than the outer rotation shaft 3a. The one end of the other connection member 32 is connected on the side closer to the other end than the inner rotation shaft 3b, and the other end of the other connection member 32 is connected on the side closer to the other end than the outer rotation shaft 3a.

The one connection member 31 and the other connection member 32 restrain a central portion of the one connection member 31 and a central portion of the other connection member 32 through a connection-restraining member 33. The connection-restraining member 33 restrains the one connection member 31 and the other connection member 32 by winding cloth, resin or metal material such that the central portion of the one connection member 31 and the central portion of the other connection member 32 do not separate from each other more than a predetermined range. It is possible to use a hinge mechanism as the connection-restraining member 33. It is preferable that the one connection member 31 and the other connection member 32 turn within a predetermined range in the interlocking manner by meshing the central portion of the one connection member 31 and the central portion of the other connection member 32 with each other. By making the connection-restraining member 33 as a timing belt or a gear mechanism, it is possible to smoothly interlock the one connection member 31 and the other connection member 32.

The one connection member 31 has a C-shape, one end of the C-shape is turnably connected through the other end portion 31b of the one inner arm 10b, and the other end of the C-shape is turnably connected through the other end portion 31a of the one outer arm 10a.

The other connection member 32 has a C-shape, one end of the C-shape is turnably connected through the one end portion 32b of the other inner arm 20b, and the other end of the C-shape is turnably connected through the one end portion 32a of the other outer arm 20a.

The one connection member 31 and the other connection member 32 are connected to each other through the connection-restraining member 33 at an intermediate position of the C-shape.

A distance between the other end portion 31b to which the one end of the one connection member 31 is connected and the one end portion 32b to which the one end of the other connection member 32 is connected, as well as a distance between the other end portion 31a to which the other end of the one connection member 31 is connected and the one end portion 32a to which the other end of the other connection member 32 is connected are varied in accordance with an extended state and a bent state caused by the one arms 10a and 10b and the other arms 20a and 20b.

According to this embodiment, three points, i.e., a connected location (other end portions 31b and 31a) between the one arms 10a and 10b of the one connection member 31, a connected location (one end portions 32b and 32a) between the other arms 20a and 20b of the other connection member 32, and a restraining location (connection-restraining member 33) between the one connection member 31 and the other connection member 32 form a triangle structure. Since a shape of the triangle is changed in accordance with the extended state and the bent state caused by the one arms 10a and 10b and the other arms 20a and 20b, it is possible to interlock the one arms 10a and 10b and the other arms 20a and 20b.

A thigh band 41 which is wound around a thigh portion is mounted on the one end of the one outer arm 10a and the one end of the one inner arm 10b. A lower thigh band 42 which is wound around a lower thigh portion is mounted on the other end of the other outer arm 20a and the other end of the other inner arm 20b.

A suspension belt 43 is placed such that it is wound between the thigh portion and the knee joint.

The one outer arm 10a includes a pin 41a for mounting the thigh band 41 on the one end, and the one inner arm 10b includes a pin 41b for mounting the thigh band 41 on the one end. The other outer arm 20a includes a pin 42a for mounting the lower thigh band 42 on the other end, and the other inner arm 20b includes a pin 42b for mounting the lower thigh band 42 on the other end.

The one arms 10a and 10b and the other arms 20a and 20b according to this embodiment are formed of resin by an integral cast. As the resin, it is possible to use thermoplastic resin or thermosetting resin. It is preferable that the thermoplastic resin is short fiber reinforced thermoplastic resin.

As the thermoplastic resin, any of the following materials is used; polyamide, polyacetal, polycarbonate, polyethylene terephthalate, polybutylene terephthalate, polyphenylene sulphide polyethersulfone, polyphenylene ether, polysulfone and liquid crystal polymer. As the short fiber, one kind or a plurality kinds of the following materials are used; carbon fiber, glass fiber,boron fiber, aramid fiber, polyethylene fiber, xyron and liquid crystal polymer. The short fiber is used as reinforcing material of the thermoplastic resin. When the short fiber reinforced thermoplastic resin is used, this is suitable for large deforming processing as compared with long fiber reinforced thermoplastic resin or woven fabric reinforced thermoplastic resin.

Although Figs. 9 show the one inner arm 10b and the other inner arm 20b, the one outer arm 10a and the other outer arm 20a also have the same configurations.

Fig. 9(a) is a front view of the one inner arm, and Fig. 9 (b) is a side view of essential portions of the one inner arm.

The one inner arm 10b is formed of resin by an integral cast. The one inner arm 10b is formed into a plate shape by integrally forming a sliding portion 11 having an arc r of 180° or more centering on the rotation shaft 3b, an arm portion 12 extending from the sliding portion 11, an extension-restricting portion 13 formed on the side of one end of the arc r of the sliding portion 11 for restricting an extending angle, and a bending-restricting portion 14 formed on the side of the other end of the arc r of the sliding portion 11 for restricting the bending angle.

It is preferable that the arc r of the sliding portion 11 is in a range of 240° to 260°, and more preferably 250°.

The extension-restricting portion 13 has an extending side abutment surface in the radial direction centering on the rotation shaft 3b, and the bending-restricting portion 14 has an bending side abutment surface in the radial direction centering on the rotation shaft 3b.

Fig. 9(c) is a front view of the other inner arm, and Fig. 9(d) is a side view of essential portions of the other inner arm.

The other inner arm 20b is formed of resin by an integral cast. The other inner arm 20b is formed into a plate shape by integrally forming a sliding portion 21 having an arc r of 180° or more centering on the rotation shaft 3b, an arm portion 22 extending from the sliding portion 21, an extension-restricting portion 23 formed on the side of one end of the arc r of the sliding portion 21 for restricting an extending angle, and a bending-restricting portion 24 formed on the side of the other end of the arc r of the sliding portion 21 for restricting the bending angle.

It is preferable that the arc r of the sliding portion 21 is in a range of 240° to 260°, and more preferably 250°.

The extension-restricting portion 23 has an extending side abutment surface in the radial direction centering on the rotation shaft 3b, and the bending-restricting portion 24 has an bending side abutment surface in the radial direction centering on the rotation shaft 3b.

The sliding portion 21 of the other arm 20b is formed by the pair of sliding surfaces 21a and 21b, the sliding portion 11 of the one inner arm 10b is placed between the pair of sliding surfaces 21a and 21b, and the sliding portion 21 of the other inner arm 20b and the sliding portion 11 of the one inner arm 10b are turnably connected to each other through the rotation shaft 3b.

According to this embodiment, since the one arms 10a and 10b and the other arms 20a and 20b are formed of resin by the integral case, a weight of the device can be reduced and fitness can be enhanced. By sliding the sliding portions 11 and 21 having the arcs r of 180° or more on each other, strength can be enhanced, and it is possible to restrict the extending motion and the bending motion with sufficient strength. In the knee/elbow joint assist device which forms the one arms 10a and 10b and the other arms 20a and 20b of resin by the integral cast, it is only necessary that the knee/elbow joint assist device includes the connection member which connects the one inner arm 10b and/or the other inner arm 20b and the one outer arm 10a and/or the other outer arm 20a to each other on the rear knee portion side. The knee/elbow joint assist device may not include the one connection member 31 and the other connection member 32 as the connection members unlike the other embodiments.

A knee/elbow joint assist device according to another embodiment of the invention will be described below.

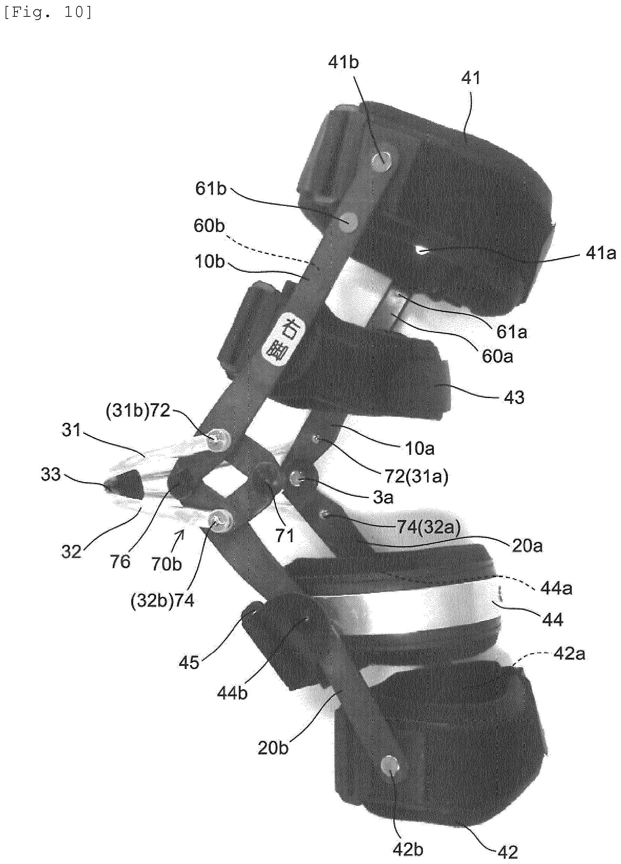

Fig. 10 is a perspective view of the knee/elbow joint assist device according to this embodiment.

The knee/elbow joint assist device of this embodiment includes the one inner arm (one arm) 10b placed on one side of an inner side of a knee joint, the other inner arm (other arm) 20b placed on the other side of the inner side of the knee joint, one outer arm (one arm) 10a placed on one of measuring sides of the knee joint, the other outer arm (other arm) 20a placed on the other measuring side of the knee joint, and the one connection member 31 and the other connection member 32 placed on the side of a front knee

The other end of the one inner arm 10b and the one end of the other inner arm 20b are turnably connected to each other through the inner link mechanism (inner turning member) 70b. The other end of the one outer arm 10a and the one end of the other outer arm 20a are turnably connected to each other through the outer rotation shaft (outer turning member) 3a.

The link mechanism described using Figs. 4 can be applied to the inner link mechanism 70b. The rotation shaft described using Figs. 9 can be applied to the outer rotation shaft 3a. The rotation shaft shown in Figs. 2 can be applied to the outer rotation shaft 3a.

The one connection member 31 and the other connection member 32 restrain a central portion of the one connection member 31 and a central portion of the other connection member 32 through a connection-restraining member 33. The connection-restraining member 33 restrains the one connection member 31 and the other connection member 32 by winding cloth, resin or metal material such that the central portion of the one connection member 31 and the central portion of the other connection member 32 do not separate from each other more than a predetermined range. It is possible to use a hinge mechanism as the connection-restraining member 33. It is preferable that the one connection member 31 and the other connection member 32 turn within a predetermined range in the interlocking manner by meshing the central portion of the one connection member 31 and the central portion of the other connection member 32 with each other. By making the connection-restraining member 33 as a timing belt or a gear mechanism, it is possible to smoothly interlock the one connection member 31 and the other connection member 32.

The one end of the one connection member 31 is turnably connected to the other end portion 31b (one inner joint 72) of the one inner arm 10b, and the other end of the one connection member 31 is turnably connected to the other end portion 31a of the one outer arm 10a on the side closer to the one end of the one outer arm 10a than the outer rotation shaft 3a. The one end of the other connection member 32 is turnably connected to the one end portion 32b (other inner joint 74) of the other inner arm 20b, and the other end of the other connection member 32 is turnably connected to the one end portion 32a of the other outer arm 20a on the side closer to the other end than the outer rotation shaft 3a.

The one connection member 31 has a C-shape, one end of the C-shape is turnably connected to the other end portion 31b of the one inner arm 10b through the one inner joint 72, and the other end of the C-shape is turnably connected to the other end portion 31a of the one outer arm 10a.

The other connection member 32 has a C-shape, one end of the C-shape is connected to the one end portion 32b of the other inner arm 20b through the other inner joint 74, and the other end of the C-shape is turnably connected to the one end portion 32a of the other outer arm 20a.

The one connection member 31 and the other connection member 32 are connected to each other through the connection-restraining member 33 at an intermediate position of the C-shape.

A distance between the other end portion 31b to which the one end of the one connection member 31 is connected and the one end portion 32b to which the one end of the other connection member 32 is connected, as well as a distance between the other end portion 31a to which the other end of the one connection member 31 is connected and the one end portion 32a to which the other end of the other connection member 32 is connected are varied in accordance with an extended state and a bent state caused by the one arms 10a and 10b and the other arms 20a and 20b.

According to this embodiment, three points, i.e., a connected location (other end portions 31b and 31a) between the one arms 10a and 10b of the one connection member 31, a connected location (one end portions 32b and 32a) between the other arms 20a and 20b of the other connection member 32, and a restraining location (connection-restraining member 33) between the one connection member 31 and the other connection member 32 form a triangle structure. Since a shape of the triangle is changed in accordance with the extended state and the bent state caused by the one arms 10a and 10b and the other arms 20a and 20b, it is possible to interlock the one arms 10a and 10b and the other arms 20a and 20b.

A thigh band 41 which is wound around a thigh portion is mounted on the one end of the one outer arm 10a and the one end of the one inner arm 10b. A lower thigh band 42 which is wound around a lower thigh portion is mounted on the other end of the other outer arm 20a and the other end of the other inner arm 20b.

A suspension belt 43 is placed such that it is wound between the thigh portion and the knee joint.

A calf 44 which is placed at a calf portion located higher than a maximum bulging portion of the calf portion is mounted on the other outer arm 20a and the other inner arm 20b. A calf band 45 placed on the side of a tibia is mounted on the other outer arm 20a and the other inner arm 20b.

The one outer arm 10a includes a pin 41a for mounting the thigh band 41 on the one end, and the one inner arm 10b includes a pin 41b for mounting the thigh band 41 on the one end. The other outer arm 20a includes a pin 42a for mounting the lower thigh band 42 on the other end, and the other inner arm 20b includes a pin 42b for mounting the lower thigh band 42 on the other end.

The thigh band 41 is provided such that it can turn with respect to the one outer arm 10a through the pin 41a and such that the thigh band 41 can turn with respect to the one inner arm 10b through the pin 41b. The lower thigh band 42 is provided such that it can turn with respect to the other outer arm 20a through the pin 42a and such that the lower thigh band 42 can turn with respect to the other inner arm 20b through the pin 42b.

One end of an inner suspension arm 60b is mounted on an inner side of the one inner arm 10b through a pin 61b. The suspension belt 43 is mounted on the other end of the inner suspension arm 60b. The inner suspension arm 60b is formed of spring material such as a leaf spring, and the inner suspension arm 60b is provided such that it elastically deforms between the one inner arm 10b and a body.

One end of an outer suspension arm 60a is mounted on an inner side of the one outer arm 10a through a connecting portion 61a. The suspension belt 43 is mounted on the other end of the outer suspension arm 60a. The outer suspension arm 60a is formed of spring material such as a leaf spring, and provided such that it elastically deforms between the one outer arm 10a and a body.

The other outer arm 20a includes a pin 44a for mounting the calf 44 and the calf band 45 on the side closer to the one end than the pin 42a.

The calf 44 and the calf band 45 are provided such that they can turn with respect to the other outer arm 20a through the pin 44a.

The other inner arm 20b includes a pin 44b for mounting the calf 44 and the calf band 45 on the side closer to the one end than the pin 42b.

The calf 44 and the calf band 45 are provided such that they can turn with respect to the other inner arm 20b through the pin 44b.

According to the knee/elbow joint assist device of this embodiment, by using the inner link mechanism 70b where the four joints 71, 72, 74 and 76 are placed, the one inner joint 72 located at a position of the one inner arm 10b moves upward (toward one end) as the knee joint moves from the bent state to the extended state, and the other inner joint 74 located at a position of the other inner arm 20b moves downward (toward other end). Therefore, a force to vertically separate is applied to an inner side of the knee joint by the inner link mechanism 70b at the time of the extending motion. Since the force to vertically separate the inner side of the knee joint is applied by the inner link mechanism 70b at the time of the extending motion, it is possible to reduce a load bearing on the knee joint, pain of the knee joint is subdued and a patient is encouraged to walk.

Shaking of the outer side of the knee joint at the time of the extending motion and the bending motion is not generated by using the single outer rotation shaft 3a. Therefore, the force to vertically separate which is applied to the inner side of the knee joint can reliably be applied.

As shown in this embodiment, the one connection member 31 and the other connection member 32 can be placed on the front side of the knee joint, i.e., on the side of a front knee.

Although the knee/elbow joint assist device described using Figs. 1 to 10 is used for the knee joint, the knee/elbow joint assist device can also similarly be applied to an elbow joint.

### [INDUSTRIAL APPLICABILITY]

The knee/elbow joint assist device of the present invention can be used for back knee such as physical therapy and polio of hemiparesis, can be used for degenerative joint such as bowleg, X leg and rheumatism, can be used for sport such as ligament injury and medial meniscus injury, and the knee/elbow joint assist device of the invention can also widely be used by operators having burden on knees, and can also be used for elbow joint.

### [EXPLANATION OF SYMBOLS]

- 10a: one outer arm
- 10b: one inner arm
- 20a: other outer arm
- 20b: other inner arm
- 31: one connection member
- 32: other connection member
- 33: connection-restraining member
- 70a: outer link mechanism
- 70b: inner link mechanism
- 71: inner front joint, outer front joint
- 72: one inner joint, one outer joint
- 73: one inner link material, one outer link material
- 74: other inner joint, other outer joint
- 75: other inner link material, other outer link material
- 76: inner rear joint, outer rear joint

## Claims

1. A knee/elbow joint assist device comprising:
a thigh band (41) suitable to be wound around a thigh portion is mounted on one end of a first arm (10a) and one end of a second arm (10b);
a lower thigh band (42) suitable to be wound around a lower thigh portion is mounted on one end of a third arm (20a) and one end of a fourth arm (20b); a first turning member (3b,70b) for turnably connecting other end of the second arm (10b) and one end of the fourth arm (20b); and
a second turning member (3a, 70a) for turnably connecting other end of the first arm (10a) and one end of the third arm (20a), in which
the second arm (10b) and the first arm (10a) compose one arm, and
the fourth arm (20b) and the third arm (20a) compose other arm, wherein
one connection member (31) and other connection member (32) are suitable to be placed on the knee/elbow joint,
the one connection member (31) turnably connects one end to other end portion of the second arm (10b), and turnably connects other end to the other end portion of the first arm (10a),
the other connection member (32) turnably connects one end to one end portion of the fourth arm (20b), and turnably connects other end to one end portion of the third arm (20a), and
a connection-restraining member (33) is configured to restrain the one connection member (31) and the other connection member (32), thereby interlocking the one arm and the other arm to each other.

2. The knee/elbow joint assist device according to claim 1, wherein
the first turning member (3b) and the second turning member (3a) are rotation shafts,
the other end portion of the second arm (10b) configured to connect to the one end of the one connection member (31) is closer to one end of the second arm (10b) than the first turning member (3b),
the one end portion of the fourth arm (20b) which connects the one end of the other connection member (32) is closer to other end of the fourth arm (20b) than the first turning member (3b),
the other end portion of the first arm (10a) which connects the other end of the one connection member (31) is closer to one end of the first arm (10a) than the second turning member (3a), and
the one end portion of the fourth arm (20b) which connects the other end of the other connection member (32) is closer to other end of the fourth arm (20b) than the second turning member (3a) .

3. The knee/elbow joint assist device according to claim 1, wherein
the first turning member (70b) and the second turning member (70a) are link mechanisms (70a,70b),
the link mechanism (70a,70b) is composed of
a front joint (71) which turnably connects other end of the one arm and one end of the other arm,
one link material (73) which is turnably providing one end through one joint on the other end side of the one arm,
other link material (75) which is turnably providing other end through other joint on the one end side of the other arm, and
a rear joint (76) which turnably connects other end of the one link material (73) and one end of the other link material (75),
the one end and the other end of the one connection member (31) are connected to each other through the one joint, and
the one end and the other end of the other connection member (32) are connected to each other through the other joint.

4. The knee/elbow joint assist device according to claim 1, wherein
the first turning member (3b) is a rotation shaft (3a,3b),
the other end portion of the second arm (10b) which connects the one end of the one connection member (31) is closer to one end of the second arm (10b) than the first turning member (3b),
the one end portion of the fourth arm (20b) which connects the one end of the other connection member (32) is closer to other end of the fourth arm (20b) than the first turning member (3b),
the second turning member (70a) is a link mechanism (70a,70b),
the link mechanism (70a,70b) is composed of
a front joint (71) which turnably connects the other end of the first arm (10a) and the one end of the third arm (20a),
one link material (73) which is turnably providing one end through one joint on the other end side of the first arm (10a),
other link material (75) which is turnably providing other end through other joint on the one end side of the third arm (20a), and
a rear joint (76) which turnably connects other end of the one link material (73) and one end of the other link material (75),
the other end of the one connection member (31) is connected through the one joint, and
the other end of the other connection member (32) is connected through the other joint.

5. The knee/elbow joint assist device according to claim 1, wherein
the first turning member (3a) is a rotation shaft (3a,3b),
the other end portion of the first arm (10a) which connects the one end of the one connection member (31) is closer to one end of the first arm (10a) than the second turning member (3a),
the one end portion of the third arm (20a) which connects the one end of the other connection member (32) is closer to other end of the third arm (20a) than the second turning member (3a),
the first turning member (70b) is a link mechanism (70a,70b),
the link mechanism (70a,70b) is composed of
a front joint (71) which turnably connects the other end of the second arm (10b) and the one end of the fourth arm (20b),
one link material (73) which is turnably providing one end through one joint on the other end side of the second arm (10b),
other link material (75) which is turnably providing other end through other joint on the one end side of the fourth arm (20b), and
a rear joint (76) which turnably connects other end of the one link material (73) and one end of the other link material (75),
the other end of the one connection member (31) is connected through the one joint, and
the other end of the other connection member (32) is connected through the other joint.

6. The knee/elbow joint assist device according to claim 2, wherein
each of the one arm and the other arm is formed of resin by an integral cast from
a sliding portion (11) having an arc of 180° or more centering on the rotation shaft (3a,3b),
an arm portion extending from the sliding portion (11),
an extension-restricting portion formed on one end side of the arc of the sliding portion (11) for restricting an extending angle, and
a bending-restricting portion (14) formed on other end side of the arc of the sliding portion (11) for restricting a bending angle,
the sliding portion (11) of one of the one arm and the other arm is formed by a pair of sliding surfaces,
the sliding portion (11) of other one of the one arm and the other arm is placed between the pair of sliding surfaces, and
the sliding portion (11) of the one of the one arm and the other arm and the sliding portion (11) of the other one of the one arm and the other arm are turnably connected to each other through the rotation shaft (3a,3b).

7. The knee/elbow joint assist device according to claim 6, wherein
the extension-restricting portion (13) formed on the one arm and the extension-restricting portion (13) formed on the other arm are extension side abutment surfaces in a radial direction centering on the rotation shaft (3a,3b), and
the bending-restricting portion (14) formed on the one arm and the bending-restricting portion (14) formed on the other arm are bending side abutment surfaces in a radial direction centering on the rotation shaft (3a,3b).

## Patentansprüche

1. Eine Vorrichtung zum Stützen des Knie-/Ellbogengelenks, aufweisend:
ein Oberschenkelband (41), das um einen Oberschenkelabschnitt gewickelt werden kann und an einem Ende eines ersten Arms (10a) sowie an einem Ende eines zweiten Arms (10b) befestigt ist;
ein Unterschenkelband (42), das um einen Unterschenkelabschnitt gewickelt werden kann und an einem Ende eines dritten Arms (20a) und an einem Ende eines vierten Arms (20b) befestigt ist;
ein erstes Drehelement (3b, 70b) zum drehbaren Verbinden des anderen Endes des zweiten Arms (10b) mit einem Ende des vierten Arms (20b);
und
ein zweites Drehelement (3a, 70a) zum drehbaren Verbinden des anderen Endes des ersten Arms (10a) mit einem Ende des dritten Arms (20a), wobei
der zweite Arm (10b) und der erste Arm (10a) einen Arm bilden, und
der vierte Arm (20b) und der dritte Arm (20a) einen anderen Arm bilden,
wobei ein Verbindungselement (31) und ein anderes Verbindungselement (32) geeignet sind, an dem Knie-/Ellbogengelenk angeordnet zu werden,
das eine Verbindungselement (31) ein Ende mit dem anderen Endabschnitt des zweiten Arms (10b) und das andere Ende mit dem anderen Endabschnitt des ersten Arms (10a) drehbar verbindet,
das andere Verbindungselement (32) ein Ende mit dem Endabschnitt des vierten Arms (20b) und das andere Ende mit einem Endabschnitt des dritten Arms (20a) drehbar verbindet, und wobei
ein Verbindungshalteelement (33) so ausgebildet ist, dass es das eine Verbindungselement (31) und das andere Verbindungselement (32) zusammenhält, wodurch der eine Arm und der andere Arm verschränkt werden.

2. Die Vorrichtung zum Stützen des Knie-/Ellbogengelenks nach Anspruch 1, wobei
das erste Drehelement (3b) und das zweite Drehelement (3a) Drehwellen darstellen,
der andere Endabschnitt des zweiten Arms (10b), ausgebildet, um mit dem einen Ende des einen Verbindungselements (31) verbunden zu werden, dem einen Ende des zweiten Arms (10b) näher ist, als das erste innere Drehelement (3b),
der eine, das eine Ende des anderen Verbindungselement (32) verbindende Endabschnitt des vierten Arms (20b) dem anderen Ende des vierten Arms (20b) näher ist, als das erste Drehelement (3b),
der andere, das andere Ende des einen Verbindungselements (31) verbindende Endabschnitt des ersten Arms (10a) dem einen Ende des ersten Arms (10a) näher ist, als das zweite äußere Drehelement (3a), und
der eine, das andere Ende des anderen Verbindungselements (32) verbindende Endabschnitt des vierten Arms (20b) dem anderen Ende des vierten Arms (20b) näher ist als das zweite Drehelement (3a).

3. Die Vorrichtung zum Stützen des Knie-/Ellbogengelenks nach Anspruch 1, wobei
das erste Drehelement (70b) und das zweite Drehelement (70a) Gelenkmechanismen (70a, 70b) darstellen, bestehend aus:
einem vorderen Gelenk (71), welches das andere Ende des einen Arms und das eine Ende des anderen Arms drehbar miteinander verbindet,
einem Verbindungsteil (73), welches an der anderen Endseite des einen Arms durch ein Gelenk mit einem drehbaren Ende versehen ist,
ein anderes Verbindungsteil (75), welches an der einen Endseite des anderen Arms durch das andere Gelenk mit einem anderen drehbaren Ende versehen ist, und ein hinteres Gelenk (76), welches das andere Ende des einen Verbindungsteils (73) mit dem einen Ende des anderen Verbindungsteils (75) drehbar verbindet,
das eine Ende und das andere Ende des einen Verbindungselements (31) durch das eine Gelenk miteinander verbunden sind, und
das eine Ende und das andere Ende des anderen Verbindungselements (32) durch das andere Gelenk miteinander verbunden sind.

4. Die Vorrichtung zum Stützen des Knie-/Ellbogengelenks nach Anspruch 1, wobei
das erste Drehelement (3b) eine Drehwelle (3a, 3b) darstellt,
der andere, das eine Ende des einen Verbindungselements (31) verbindende Endabschnitt des zweiten Arms (10b) dem einen Ende des zweiten Arms (10b) näher ist, als das erste Drehelement (3b),
der eine, das eine Ende des anderen Verbindungselements (32) verbindende Endabschnitt des vierten Arms (20b) dem anderen Ende des vierten Arms (20b) näher ist, als das erste Drehelement (3b),
das zweite Drehelement (70a) einen Gelenkmechanismus (70a, 70b) darstellt, wobei
der Gelenkmechanismus (70a, 70b) folgende Komponenten aufweist:
ein vorderes Gelenk (71), welches das andere Ende des ersten Arms (10a) mit dem einen Ende des dritten Arms (20a) drehbar verbindet,
ein Verbindungsteil (73), welches an der anderen Endseite des ersten Arms (10a) durch ein Gelenk mit einem drehbaren Ende versehen ist,
ein anderes Verbindungsteil (75), welches an der einen Endseite des dritten Arms (20a) durch ein anderes Gelenk mit einem drehbaren Ende versehen ist, und
ein hinteres Gelenk (76), welches das andere Ende des einen Verbindungsteils (73) mit einem Ende des anderen Verbindungsteils (75) drehbar verbindet, wobei
das andere Ende des einen Verbindungselements (31) durch das eine Gelenk verbunden ist, und
das andere Ende des anderen Verbindungselements (32) durch das andere Gelenk verbunden ist.

5. Die Vorrichtung zum Stützen des Knie-/Ellbogengelenks nach Anspruch 1, wobei
das erste Drehelement (3a) eine Drehwelle (3a, 3b) darstellt,
der andere, das eine Ende des einen Verbindungselements (31) verbindende Endabschnitt des ersten Arms (10a) dem einen Ende des ersten Arms (10a) näher ist, als das zweite Drehelement (3a),
der eine, das eine Ende des anderen Verbindungselements (32) verbindende Endabschnitt des dritten Arms (20a) dem anderen Ende des dritten Arms (20a) näher ist, als das zweite Drehelement (3a),
das erste Drehelement (70b) einen Gelenkmechanismus (70a, 70b) darstellt, wobei
der Gelenkmechanismus (70a, 70b) folgende Komponenten aufweist:
ein vorderes Gelenk (71), welches das andere Ende des zweiten Arms (10b) und das eine Ende des vierten Arms (20b) drehbar miteinander verbindet,
ein Verbindungsteil (73), welches an der anderen Endseite des zweiten Arms (10b) durch ein Gelenk mit einem drehbaren Ende versehen ist,
ein anderes Verbindungsteil (75), welches an der einen Endseite des vierten Arms (20b) durch das andere Gelenk mit einem anderen drehbaren Ende versehen ist, und
ein hinteres Gelenk (76), welches das andere Ende des einen Verbindungsteils (73) und ein Ende des anderen Verbindungsteils (75) drehbar verbindet, wobei
das andere Ende des einen Verbindungselements (31) durch das eine Gelenk verbunden ist, und
das andere Ende des anderen Verbindungselements (32) durch das andere Gelenk verbunden ist.

6. Die Vorrichtung zum Stützen des Knie-/Ellbogengelenks nach Anspruch 2, wobei
sowohl der eine Arm als auch der andere Arm durch einen integralen Guss aus Harz gebildet ist, mit
einem einen Bogen von 180° oder mehr aufweisenden, auf der Drehwelle (3a, 3b) zentrierten Gleitabschnitt (11),
einem Armabschnitt, der sich von dem Gleitabschnitt (11) aus erstreckt,
einem Streckungsbegrenzungsabschnitt, der an einer Endseite des Bogens des Gleitabschnitts (11) ausgebildet ist, um einen Streckungswinkel zu begrenzen, und
einen Beugungsbegrenzungsabschnitt (14), der an der anderen Endseite des Bogens des Gleitabschnitts (11) ausgebildet ist, um einen Beugungswinkel zu begrenzen,
der Gleitabschnitt (11) des einen Arms oder des anderen Arms durch ein Paar von Gleitflächen gebildet ist,
der Gleitabschnitt (11) des anderen des einen Arms oder des anderen Arms zwischen dem Paar von Gleitflächen angeordnet ist, und
der Gleitabschnitt (11) des einen Arms oder des anderen Arms mit dem Gleitabschnitt (11) des anderen des einen Arms oder des anderen Arms durch die Drehwelle (3a, 3b) drehbar verbunden ist.

7. Die Vorrichtung zum Stützen des Knie-/Ellbogengelenks nach Anspruch 6, wobei
der an dem einen Arm ausgebildete Streckungsbegrenzungsabschnitt (13) und der an dem anderen Arm ausgebildete Streckungsbegrenzungsabschnitt (13) in einer radialen Richtung streckungsseitige, auf der Drehwelle (3a, 3b) zentrierte Anlageflächen darstellen,
und
der an dem einen Arm ausgebildete Beugungsbegrenzungsabschnitt (14) und der an dem anderen Arm ausgebildete Beugungsbegrenzungsabschnitt (14) in einer radialen Richtung beugungsseitige, auf der Drehwelle (3a, 3b) zentrierte Anlageflächen darstellen.

## Revendications

1. Dispositif d'assistance pour une articulation du genou/coude comportant :
une bande de cuisse (41) adaptée pour être enroulée autour d'une partie de cuisse, qui est monté sur une extrémité d'un premier bras (10a) et une extrémité d'un deuxième bras (10b) ;
une bande de cuisse inférieure (42) adaptée pour être enroulée autour d'une partie de cuisse inférieure, qui est monté sur une extrémité d'un troisième bras (20a) et une extrémité d'un quatrième bras (20b) ;
un premier élément rotatif (3b, 70b) pour lier en rotation l'autre extrémité du deuxième bras (10b) et une extrémité du quatrième bras (20b) ; et
un second élément rotatif (3a, 70a) pour lier en rotation l'autre extrémité du premier bras (10a) et une extrémité du troisième bras (20a), dans lequel
le deuxième bras (10a) et le premier bras (10a) composent un bras, et
le quatrième bras (20b) et le troisième bras (20a) composent l'autre bras, dans lequel
un élément de liaison (31) et un autre élément de liaison (32) sont adaptés pour être placés sur l'articulation du genou/coude ;
l'élément de liaison (31) lie en rotation une extrémité à une autre partie d'extrémité du deuxième bras (10b), et lie en rotation une autre extrémité à l'autre partie d'extrémité du premier bras (10a),
l'autre élément de liaison (32) lie en rotation une extrémité à une autre partie d'extrémité du quatrième bras (20b), et lie en rotation une autre extrémité à une partie d'extrémité du troisième bras (20a), et
un élément de retenue de liaison (33) est configuré pour retenir l'élément de liaison (31) et l'autre élément de liaison (32), en interverrouillant ainsi mutuellement l'un et l'autre bras.

2. Dispositif d'assistance pour une articulation du genou/coude selon la revendication 1, dans lequel
le premier élément rotatif (3b) et le second élément rotatif (3a) sont des axes de rotation,
l'autre partie d'extrémité du deuxième bras (10b) configurée pour être liée à l'extrémité de l'élément de liaison (31) est plus près d'une extrémité du deuxième bras (10b) que le premier élément rotatif (3b),
la partie d'extrémité du quatrième bras (20b) qui relie l'extrémité de l'autre élément de liaison (32) est plus près d'une autre extrémité du quatrième bras (20b) que le premier élément rotatif (3b),
l'autre partie d'extrémité du premier bras (10a) qui relie l'autre extrémité de l'élément de liaison (31) est plus près d'une extrémité du premier bras (10a) que le second élément rotatif (3a), et
la partie d'extrémité du quatrième bras (20b) qui relie l'autre extrémité de l'autre élément de liaison (32) est plus près d'une autre extrémité du quatrième bras (20b) que le second élément rotatif (3a).

3. Dispositif d'assistance pour une articulation du genou/coude selon la revendication 1, dans lequel
le premier élément rotatif (70b) et le second élément rotatif (70a) sont des mécanismes de liaison (70a, 70b),
le mécanisme de liaison (70a, 70b) est composé de :
une articulation avant (71) qui lie en rotation une autre extrémité du bras et une extrémité de l'autre bras,
un matériau de liaison (73) qui assure la rotation d'une extrémité par l'intermédiaire d'une articulation sur l'autre côté d'extrémité du bras,
un autre matériau de liaison (75) qui assure la rotation d'une autre extrémité par l'intermédiaire d'une autre articulation sur l'autre côté d'extrémité de l'autre bras, et
une articulation arrière (76) qui lie en rotation une autre extrémité du matériau de liaison (73) et une extrémité de l'autre matériau de liaison (75),
l'une et l'autre extrémité de l'élément de liaison (31) sont mutuellement liées par l'intermédiaire de l'articulation, et
l'une et l'autre extrémité de l'autre élément de liaison (32) sont mutuellement liées par l'intermédiaire de l'autre articulation.

4. Dispositif d'assistance pour une articulation du genou/coude selon la revendication 1, dans lequel
le premier élément rotatif (3b) est un axe de rotation (3a, 3b),
l'autre partie d'extrémité du deuxième bras (10b) qui relie l'extrémité de l'élément de liaison (31) est plus près d'une extrémité du deuxième bras (10b) que le premier élément rotatif (3b),
la partie d'extrémité du quatrième bras (20b) qui relie l'extrémité de l'autre élément de liaison (32) est plus près d'une autre extrémité du quatrième bras (20b) que le premier élément rotatif (3b),
le second élément rotatif (70a) est un mécanisme de liaison (70a, 70b),
le mécanisme de liaison (70a, 70b) est composé de :
une articulation avant (71) qui lie en rotation l'autre extrémité du premier bras (10a) et l'extrémité du troisième bras (20a),
un matériau de liaison (73) qui assure la rotation d'une extrémité par l'intermédiaire d'une articulation sur l'autre côté d'extrémité du premier bras (10a),
un autre matériau de liaison (75) qui assure la rotation d'une autre extrémité par l'intermédiaire d'une autre articulation sur l'autre côté d'extrémité du troisième bras (20a), et
une articulation arrière (76) qui lie en rotation une autre extrémité du matériau de liaison (73) et une extrémité de l'autre matériau de liaison (75),
l'autre extrémité du second élément de liaison (31) est liée par l'intermédiaire de l'articulation, et
l'autre extrémité de l'autre élément de liaison (32) est liée par l'intermédiaire de l'autre articulation.

5. Dispositif d'assistance pour une articulation du genou/coude selon la revendication 1, dans lequel
le premier élément rotatif (3a) est un axe de rotation (3a, 3b),
l'autre partie d'extrémité du premier bras (10a) qui relie l'extrémité de l'élément de liaison (31) est plus près d'une extrémité du premier bras (10a) que l'élément rotatif (3a), et
la partie d'extrémité du troisième bras (20a) qui relie l'extrémité de l'autre élément de liaison (32) est plus près d'une autre extrémité du troisième bras (20a) que le second élément rotatif (3a),
le premier élément rotatif (70b) est un mécanisme de liaison (70a, 70b),
le mécanisme de liaison (70a, 70b) est composé de :
une articulation avant (71) qui lie en rotation l'autre extrémité du deuxième bras (10b) et l'extrémité du quatrième bras (20b),
un matériau de liaison (73) qui assure la rotation d'une extrémité par l'intermédiaire d'une articulation sur l'autre côté d'extrémité du deuxième bras (10b),
un autre matériau de liaison (75) qui assure la rotation d'une autre extrémité par l'intermédiaire d'une autre articulation sur le côté d'extrémité du quatrième bras (20b), et
un joint arrière (76) qui lie en rotation une autre extrémité du matériau de liaison (73) et une extrémité de l'autre matériau de liaison (75),
l'autre extrémité de l'élément de liaison (31) est liée par l'intermédiaire de l'articulation, et
l'autre extrémité de l'autre élément de liaison (32) est liée par l'intermédiaire de l'autre articulation.

6. Dispositif d'assistance pour une articulation du genou/coude selon la revendication 2, dans lequel
l'un et l'autre bras sont chacun formés en résine par une pièce moulée d'un seul tenant à partir de :
une partie coulissante (11) ayant un arc de 180° ou plus se centrant sur l'axe de rotation (3a, 3b),
une partie de bras s'étendant à partir de la partie coulissante (11),
une partie de restriction d'extension formée sur un côté d'extrémité de l'arc de la partie coulissante (11) pour restreindre un angle d'extension, et
une partie de restriction de flexion (14) formée sur l'autre côté d'extrémité de l'arc de la partie coulissante (11) pour restreindre un angle de flexion,
la partie coulissante (11) de l'un ou l'autre bras est formée par une paire de surfaces de coulissement,
la partie coulissante (11) de l'autre bras est placée entre la paire de surfaces de coulissement, et
la partie coulissante (11) de l'un ou l'autre bras et la partie coulissante (11) de l'autre bras sont liées en rotation l'une à l'autre par l'axe de rotation (3a, 3b).

7. Dispositif d'assistance pour une articulation du genou/coude selon la revendication 6, dans lequel
la partie de restriction d'extension (13) formée sur le bras et la partie de restriction d'extension (13) formée sur l'autre bras sont des surfaces de butée latérale d'extension dans une direction se centrant sur l'axe de rotation (3a, 3b), et
la partie de restriction de flexion (14) formée sur le bras et la partie de restriction de flexion (14) formée sur l'autre bras sont des surfaces de butée latérale de flexion dans une direction radiale se centrant sur la tige de rotation (3a, 3b).
